# EUROPEAN PATENT SPECIFICATION

(11) **EP 2 336 264 B1**
(45) Date of publication and mention of the grant of the patent: **07.08.2019**
(21) Application number: 10014887.3
(22) Date of filing: 26.06.2006
(51) Int. Cl.: C09K 5/04, A61F 7/00

(54) **Compositions containing fluorine substituted olefins**
Zusammensetzungen mit fluorsubstituierten Olefinen
Compositions contenant des oléfines substituées de fluorine

(30) Priority: 24.06.2005 US 693853 P
(43) Date of publication of application: 22.06.2011
(62) Divisional of application: 06785612.0
(73) Proprietor: Honeywell International Inc., Morris Plains, NJ 07950 (US)
(72) Inventor: Singh, Rajiv R., Morristown, NJ 07962-2245 (US); Pham, Hang T., Morristown, NJ 07962-2245 (US)
(74) Representative: Crooks, Elizabeth Caroline

(56) References cited:
- WO-A2-2005/008819
- US-A- 5 809 787
- US-A- 5 860 292
- US-A- 5 941 243
- US-A1- 2004 064 170
- US-A1- 2004 119 047

## Description

### FIELD OF THE INVENTION

This invention relates to uses and compositions having utility in evaporative cooling.

### BACKGROUND

Fluorocarbon based fluids have found widespread use in many commercial and industrial applications, including as the working fluid in systems such as air conditioning, heat pump and refrigeration systems, as aerosol propellants, as blowing agents, as heat transfer media, and as gaseous dielectrics. Because of certain suspected
environmental problems, including the relatively high global warming potentials, associated with the use of some of the compositions that have heretofore been used in these applications, it has become increasingly desirable to use fluids having low or even zero ozone depletion potential, such as hydrofluorocarbons ("HFCs"). Thus, the use of fluids that do not contain chlorofluorocarbons ("CFCs") or hydrochlorofluorocarbons ("HCFCs") is desirable. Furthermore, some HFC fluids may have relatively high global warming potentials associated therewith, and it is desirable to use hydrofluorocarbon or other fluorinated fluids having as low global warming potentials as possible while maintaining the desired performance in use properties. Additionally, the use of single component fluids or azeotrope-like mixtures, which do not substantially fractionate on boiling and evaporation, is desirable in certain circumstances.

Certain fluorocarbons have been a preferred component in many heat exchange fluids, such as refrigerants, for many years in many applications. For, example, fluoroalkanes, such as chlorofluoromethane and chlorofluoroethane derivatives, have gained widespread use as refrigerants in applications including air conditioning and heat pump applications owing to their unique combination of chemical and physical properties. Many of the refrigerants commonly utilized in vapor compression systems are either single components fluids or azeotropic mixtures.

As suggested above, concern has been increasing in recent years about potential damage to the earth's atmosphere and climate, and certain chlorine-based compounds have been identified as particularly problematic in this regard. The use of chlorine-containing compositions (such as chlorofluorocarbons (CFC's), hydrochlorofluorocarbons (HCF's) and the like) as the working fluid in heat transfer systems, such as in refrigeration and air-conditioning systems, has become disfavored because of the ozone-depleting properties associated with many of such compounds. There has thus been an increasing need for new fluorocarbon and hydrofluorocarbon compounds and compositions that are attractive alternatives to the compositions heretofore used in these and other applications. For example, it has become desirable to retrofit chlorine-containing refrigeration systems by replacing chlorine-containing refrigerants with non-chlorine-containing refrigerant compounds that will not deplete the ozone layer, such as hydrofluorocarbons (HFC's). Industry in general and the heat transfer industry in particular are continually seeking new fluorocarbon based mixtures that offer alternatives to, and are considered environmentally safer substitutes for, CFCs and HCFCs. It is generally considered important, however, at least with respect to heat transfer fluids, that any potential substitute must also possess those properties present in many of the most widely used fluids, such as excellent heat transfer properties, chemical stability, low- or no- toxicity, non-flammability and/or lubricant compatibility, among others.

Applicants have come to appreciate that lubricant compatibility is of particular importance in many of applications. More particularly, it is highly desirable for refrigeration fluids to be compatible with the lubricant utilized in the compressor unit, used in most refrigeration systems. Unfortunately, many non-chlorine-containing refrigeration fluids, including HFC's, are relatively insoluble and/or immiscible in the types of lubricants used traditionally with CFC's and HFC's, including, for example, mineral oils, alkylbenzenes or poly(alpha-olefins). In order for a refrigeration fluid-lubricant combination to work at a desirable level of efficiently within a compression refrigeration, air-conditioning and/or heat pump system, the lubricant should be sufficiently soluble in the refrigeration liquid over a wide range of operating temperatures. Such solubility lowers the viscosity of the lubricant and allows it to flow more easily throughout the system. In the absence of such solubility, lubricants tend to become lodged in the coils of the evaporator of the refrigeration, air-conditioning or heat pump system, as well as other parts of the system, and thus reduce the system efficiency.

With regard to efficiency in use, it is important to note that a loss in refrigerant thermodynamic performance or energy efficiency may have secondary environmental impacts through increased fossil fuel usage arising from an increased demand for electrical energy.

Furthermore, it is generally considered desirably for CFC refrigerant substitutes to be effective without major engineering changes to conventional vapor compression technology currently used with CFC refrigerants.

Flammability is another important property for many applications. That is, it is considered either important or essential in many applications, including particularly in heat transfer applications, to use compositions which are non-flammable. Thus, it is frequently beneficial to use in such compositions compounds which are nonflammable. As used herein, the term "nonflammable" refers to compounds or compositions which are determined to be nonflammable as determined in accordance with ASTM standard E-681, dated 2002, which is incorporated herein by reference. Unfortunately, many HFC's which might otherwise be desirable for used in refrigerant compositions are not nonflammable. For example, the fluoroalkane difluoroethane (HFC-152a) and the fluoroalkene 1,1,1-trifluorpropene (HFO-1243zf) are each flammable and therefore not viable for use in many applications.

Higher fluoroalkenes, that is fluorine-substituted alkenes having at least five carbon atoms, have been suggested for use as refrigerants. U.S. Patent No. 4,788,352 - Smutny is directed to production of fluorinated C₅ to C₈ compounds having at least some degree of unsaturation. The Smutny patent identifies such higher olefins as being known to have utility as refrigerants, pesticides, dielectric fluids, heat transfer fluids, solvents, and intermediates in various chemical reactions. (See column 1, lines 11-22).

While the fluorinated olefins described in Smutny may have some level of effectiveness in heat transfer applications, it is believed that such compounds may also have certain disadvantages. For example, some of these compounds may tend to attack substrates, particularly general-purpose plastics such as acrylic resins and ABS resins. Furthermore, the higher olefinic compounds described in Smutny may also be undesirable in certain applications because of the potential level of toxicity of such compounds which may arise as a result of pesticide activity noted in Smutny. Also, such compounds may have a boiling point which is too high to make them useful as a refrigerant in certain applications.

Bromofluoromethane and bromochlorofluoromethane derivatives, particularly bromotrifluoromethane (Halon 1301) and bromochlorodifluoromethane (Halon 1211) have gained widespread use as fire extinguishing agents in enclosed areas such as airplane cabins and computer rooms. However, the use of various halons is being phased out due to their high ozone depletion. Moreover, as halons are frequently used in areas where humans are present, suitable replacements must also be safe to humans at concentrations necessary to suppress or extinguish fire.

Applicants have thus come to appreciate a need for compositions, and particularly heat transfer compositions, fire extinguishing/suppression compositions, blowing agents, solvent compositions, and compatibilizing agents, that are potentially useful in numerous applications, including vapor compression heating and cooling systems and methods, while avoiding one or more of the disadvantages noted above.

US 2004/119047 relates to the use of pentafluoropropene (HFO-1225) and tetrafluoropropene (HFO-1234) in refrigeration, as blowing agents, as aerosol propellants, as solvent compositions, and as fire extinguishing and suppressing agents.

US 5 941 243 relates to an apparatus for topical anesthesia by evaporative cooling with ethyl chloride (chloroethane).

US 5 860 292 relates to an inflatable thermal blanket for convectively and evaporatively cooling a patient.

WO 2005/008819 relates a cooling system for an electrochemical reactor that generates electrical energy and heat energy.

US 2004/064170 relates to a device for rapidly cooling the body of a patient.

US 5 809 787 relates to a method of cooling food in a flexible synthetic pouch to a temperature below 10°C.

### SUMMARY

Applicants have found that the above-noted need, and other needs, can be satisfied by the subject matter as defined in the attached claims. That is, the use as an evaporative cooling agent of a composition comprising 1,1,1-trifluoro-3-chloropropene (HFCO-1233zd) which is a fluoroalkene having the Formula I:

XCF_{z}R_{3-z} (I)

where X is a C2 to C5 unsaturated, substituted or unsubstituted, radical, R is independently Cl, F, Br, I or H, and z is 1 to 3,
comprising bringing the composition into contact, either directly or indirectly, with a body to be cooled and thereafter permitting the composition to evaporate or boil while in contact with said body, thereby absorbing heat from said body, wherein the composition is in liquid form and is sprayed onto the body to be cooled.

The term HFCO-1233zd is used herein generically to refer to 1,1,1-trifluo-3,chloropropene, independent of whether it is the cis- or trans- form. The terms "cisHFCO-1233zd" and "transHFCO-1233zd" are used herein to describe the cis- and trans- forms of 1, 1, 1-trifluo,3-chlororopropene, respectively. The term "HFCO-1233zd" therefore includes within its scope cisHFCO-1233zd, transHFCO-1233zd, and all combinations and mixtures of these.

### DETAILED DESCRIPTION OF PREFERRED EMBODIMENTS

### THE COMPOSITIONS

The preferred embodiments of the present invention are directed to the use of compositions comprising 1,1,1-trifluoro-3-chloropropene (HFCO-1233zd). The fluoroalkene compounds for use in the present invention are sometimes referred to herein for the purpose of convenience as hydrofluoro-olefins or "HFOs" if they contain at least one hydrogen. Although it is contemplated that the HFOs of the present invention may contain two carbon-carbon double bonds, such compounds at the present time are not considered to be preferred. For HFOs which also contain at least one chlorine atom, the designation HFCO is sometimes used herein

As mentioned above, the present compositions comprise 1,1,1-trifluoro-3-chloropropene (HFCO-1233zd) which is in accordance with Formula I. 1,1,1-trifluoro-3-chloropropene (HFCO-1233zd) is also in accordance with Formula II below: 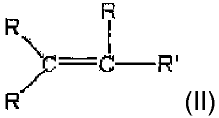
where each R is independently Cl, F, Br, I or H,
R' is (CR₂)ₙY,
Y is CRF₂
and n is 0, 1, 2 or 3, preferably 0 or 1, it being generally preferred however that when Br is present in the compound there is no hydrogen in the compound. In certain embodiments, Br is not present in the compound.

Preferably, Y is CF₃, n is 0 or 1 (most preferably 0) and at least one of the remaining Rs is F, and preferably no R is Br or when Br is present, there is no hydrogen in the compound.

Applicants believe that, in general, the compounds of the above identified Formulas I and II are generally effective and exhibit utility in evaporative cooling. However, applicants have surprisingly and unexpectedly found that certain of the compounds having a structure in accordance with the formulas described above exhibit a highly desirable low level of toxicity compared to other of such compounds. As can be readily appreciated, this discovery is of potentially enormous advantage and benefit for the formulation of not only refrigerant compositions, but also any and all compositions which would otherwise contain relatively toxic compounds satisfying the formulas described above. More particularly, applicants believe that a relatively low toxicity level is associated with compounds of Formula II, preferably wherein Y is CF₃, n is 0 or 1, wherein at least one R on the unsaturated terminal carbon is H, and at least one of the remaining Rs is F or Cl. Applicants believe also that all structural, geometric and stereoisomers of such compounds are effective and of beneficially low toxicity.

Preferred compounds comprise one or more comprises a C3 or C4 HFO, preferably a C3 HFO, and preferably a compound accordance with Formula I in which X is a halogen substituted C₃ alkylene and z is 3. Preferably X is fluorine and/or chlorine substituted C₃ alkylene, with the following C₃ alkylene radicals being preferred in certain embodiments:

-CH=CF-CH₃

-CF=CH-CH₃

-CH₂-CF=CH₂

-CH₂-CH=CFH,

Such embodiments therefore comprise the following preferred compounds: CF₃- CH=CF-CH₃; CF₃-CF=CH-CH₃; CF₃-CH₂-CF=CH₂; CF₃-CH₂-CH=CFH; and combinations of these with one another and/or with other compounds in accordance with Formula I.

Preferably, the compound comprises a C3 or C4 HFCO, preferably a C3 HFCO, and more preferably a compound in accordance with Formula II in which Y is CF₃, n is 0, at least one R on the unsaturated terminal carbon is H, and at least one of the remaining Rs is Cl. HFCO1233 is an example of such a preferred compound.

Preferably, especially embodiments which comprise the low toxicity compounds described above, n is zero.

The present compositions are believed to possess properties that are advantageous for a number of important reasons. For example, applicants believe, based at least in part on mathematical modeling, that the fluoroolefins for use in the present invention will not have a substantial negative affect on atmospheric chemistry, being negligible contributors to ozone depletion in comparison to some other halogenated species. The preferred compositions thus have the advantage of not contributing substantially to ozone depletion. The preferred compositions also do not contribute substantially to global warming compared to many of the hydrofluoroalkanes presently in use.

Of course other compounds and/or components that modulate a particular property of the compositions (such as cost for example) may also be included in the present compositions, and the presence of all such compounds and components is within the broad scope of the invention.

In certain preferred forms, compositions of the present invention have a Global Warming Potential (GWP) of not greater than about 1000, more preferably not greater than about 500, and even more preferably not greater than about 150. In certain embodiments, the GWP of the present compositions is not greater than about 100 and even more preferably not greater than about 75. As used herein, "GWP" is measured relative to that of carbon dioxide and over a 100 year time horizon, as defined in "The Scientific Assessment of Ozone Depletion, 2002, a report of the World Meteorological Association's Global Ozone Research and Monitoring Project".

In certain preferred forms, the present compositions also preferably have an Ozone Depletion Potential (ODP) of not greater than 0.05, more preferably not greater than 0.02 and even more preferably about zero. As used herein, "ODP" is as defined in "The Scientific Assessment of Ozone Depletion, 2002, A report of the World Meteorological Association's Global Ozone Research and Monitoring Project".

The amount of the Formula I compounds contained in the present compositions can vary widely, depending the particular application, and compositions containing more than trace amounts and less than 100% of the compound are within broad the scope of the present invention. Moreover, the compositions for use in the present invention can be azeotropic, azeotrope-like or non-azeotropic. Preferably, the present compositions comprise Formula I compounds, preferably HFO-1234 and more preferably HFO-1234ze and/or HFO-1234yf, preferably HFO-1234ze and/or HFO-1234yf, in amounts from about 5% by weight to about 99% by weight, and even more preferably from about 5% to about 95%. Many additional compounds or components, including lubricants, stabilizers, metal passivators, corrosion inhibitors, flammability suppressants, and other compounds and/or components that modulate a particular property of the compositions (such as cost for example) may be included in the present compositions, and the presence of all such compounds and components is within the broad scope of the invention. Preferably, the present compositions include, in addition to the compounds of formula I (including particularly HFO-1234ze and/or HFO-1234yf, one or more of the following:
Trichlorofluoromethane (CFC-11)
Dichlorodifluoromethane (CFC-12)
Difluoromethane (HFC-32)
Pentafluoroethane (HFC-125)
1,1,2,2-tetrafluoroethane (HFC-134)
1,1,1,2-Tetrafluoroethane (HFC-134a)
Difluoroethane (HFC-152a)
1,1,1,2,3,3,3-Heptafluoropropane (HFC-227ea)
1,1,1,3,3,3-hexafluoropropane (HFC-236fa)
1,1,1,3,3-pentafluoropropane (HFC-245fa)
1,1,1,3,3-pentafluorobutane (HFC-365mfc)
water
CO₂

The relative amount of any of the above noted compounds, as well as any additional components which may be included in present compositions, can vary widely within the general broad scope of the present invention according to the particular application for the composition, and all such relative amounts are considered to be within the scope hereof.

Accordingly, applicants have recognized that certain compositions described herein can be used to great advantage in a number of applications. It is believed that those of skill in the art will be readily able to adapt the present compositions for use in any and all such applications without undue experimentation.

### HEAT TRANSFER COMPOSITIONS

The compositions for use in the present invention are generally adaptable for use as evaporative cooling agents.

In connection with evaporative cooling applications, the compositions for use in the present invention are brought in contact, either directly or indirectly, with a body to be cooled and thereafter permitted to evaporate or boil while in such contact, with the preferred result that the boiling gas in accordance with the present composition absorbs heat from the body to be cooled. In such applications it may be preferred to utilize the compositions, preferably in liquid form, by spraying or otherwise applying the liquid to the body to be cooled. In other evaporative cooling applications, it may be preferred to permit a liquid composition in accordance with the present intention to escape from a relatively high pressure container into a relatively lower pressure environment wherein the body to be cooled is in contact, either directly or indirectly, with the container enclosing the liquid composition of the present invention, preferably without recovering or recompressing the escaped gas. One particular application for this type of embodiment is the self-cooling of a beverage, food item, novelty item or the like. Previous to the invention described herein, prior compositions, such as HFC-152a and HFC-134a were used for such applications. However, such compositions have recently been looked upon negatively in such application because of the negative environmental impact caused by release of these materials into the atmosphere. For example, the United States EPA has determined that the use of such prior chemicals in this application is unacceptable due to the high global warming nature of these chemicals and the resulting detrimental effect on the environment that may result from their use. The compositions for use in the present invention should have a distinct advantage in this regard due to their low global warming potential and low ozone depletion potential, as described herein. Additionally, the present compositions are expected to also find substantial utility in connection with the cooling of electrical or electronic components, either during manufacture or during accelerated lifetime testing. In a accelerated lifetime testing, the component is sequentially heated and cooled in rapid succession to simulate the use of the component. Such uses would therefore be of particular advantage in the semiconductor and computer board manufacturing industry. Another advantage of the present compositions in this regard is they are expected to exhibit as contagious electrical properties when used in connection with such applications. Another evaporative cooling application comprises methods for temporarily causing a discontinuation of the flow of fluid through a conduit. Preferably, such methods would include contacting the conduit, such as a water pipe through which water is flowing, with a liquid composition for use in the present invention and allowing the liquid composition to evaporate while in contact with the conduit so as to freeze liquid contained therein and thereby temporarily stop the flow of fluid through the conduit. Such methods have distinct advantage in connection with enabling the service or other work to be performed on such conduits, or systems connected to such conduits, at a location downstream of the location at which the present composition is applied.

Although it is contemplated that the compositions for use in the present invention may include the compounds described herein in widely ranging amounts, it is generally preferred that refrigerant compositions comprise compound(s) in accordance with Formula I, more preferably in accordance with Formula II, and even more preferably HFO-1234 (including HFO-1234ze and HFO-1234yf), in an amount that is at least about 50% by weight, and even more preferably at least about 70 % by weight, of the composition. In certain embodiments, it is preferred that the heat transfer compositions comprise transHFO-1234ze. In certain preferred embodiments, it is preferred that the heat transfer compositions comprise at least about 80%, and even more preferably at least about 90% by weight of HFO-1234, and even more preferably HFO-1234yf and/or HFO-1234ze. The heat transfer compositions comprise in certain embodiments a combination of cisHFO-1234ze and transHFO1234ze, preferably in a cis:trans weight ratio of from about 1:99 to about 10:99, more preferably from about 1:99 to about 5:95, and even more preferably from about 1:99 to about 3:97.

The relative amount of the hydrofluoroolefin used in accordance with the present invention is preferably selected to produce a heat transfer fluid which has the required heat transfer capacity, particularly refrigeration capacity, and preferably is at the same time non-flammable. As used herein, the term non-flammable refers to a fluid which is non-flammable in all proportions in air as measured by ASTM E-681.

The compositions for use in the present invention may include other components for the purpose of enhancing or providing certain functionality to the composition, or in some cases to reduce the cost of the composition. For example, refrigerant compositions include a lubricant, generally in amounts of from about 30 to about 50 percent by weight of the composition. Furthermore, the present compositions may also include a co-refrigerant, or compatibilzer, such as propane, for the purpose of aiding compatibility and/or solubility of the lubricant. Such compatibilizers, including propane, butanes and pentanes, are preferably present in amounts of from about 0.5 to about 5 percent by weight of the composition. Combinations of surfactants and solubilizing agents may also be added to the present compositions to aid oil solubility, as disclosed by U.S. Patent No. 6,516,837. Commonly used refrigeration lubricants such as Polyol Esters (POEs) and Poly Alkylene Glycols (PAGs), PAG oils, silicone oil, mineral oil, alkyl benzenes (ABs) and poly(alphaolefin) (PAO) that are used in refrigeration machinery with hydrofluorocarbon (HFC) refrigerants may be used with the refrigerant compositions described herein. Commercially available mineral oils include Witco LP 250 (registered trademark) from Witco, Zerol 300 (registered trademark) from Shrieve Chemical, Sunisco 3GS from Witco, and Calumet R015 from Calumet. Commercially available alkyl benzene lubricants include Zerol 150 (registered trademark). Commercially available esters include neopentyl glycol dipelargonate, which is available as Emery 2917 (registered trademark) and Hatcol 2370 (registered trademark). Other useful esters include phosphate esters, dibasic acid esters, and fluoroesters. In some cases, hydrocarbon based oils are have sufficient solubility with the refrigerant that is comprised of an iodocarbon, the combination of the iodocarbon and the hydrocarbon oil might more stable than other types of lubricant. Such combination may therefore be advantageous. Preferred lubricants include polyalkylene glycols and esters. Polyalkylene glycols are highly preferred in certain embodiments because they are currently in use in particular applications such as mobile air-conditioning. Of course, different mixtures of different types of lubricants may be used.

In certain preferred embodiments, the heat transfer composition comprises from about 10% to about 95 % by weight of a compound of Formula I, more preferably a compound of Formula II, and even more preferably one or more HFO-1234 compounds, and from about 5% to about 90% by weight of an adjuvant, particular in certain embodiments a co-refrigerant (such as HFC-152, HFC-125 and/or CF₃I). The use of the term co-refrigerant is not intended for use herein in a limiting sense regarding the relative performance of the compound of Formula I compounds, but is instead used to identify other components of the refrigerant composition generally that contribute to the desirable heat transfer characteristics of the composition for a desired application. In certain of such embodiments the co-refrigerant comprises, and preferably consists essentially of, one or more HFCs and/or one or more fluoroiodo C1 - C3 compounds, such as trifluroiodomethane, and combinations of these with each other and with other components.

In preferred embodiments in which the co-refrigerant comprises HFC, preferably HFC-125 the composition comprises HFC in an amount of from about 50% by weight to about 95% by weight of the total heat transfer composition, more preferably from about 60% by weight to about 90% by weight, and even more preferably of from about 70% to about 90% by weight of the composition. In such embodiments the compounds preferably comprise, and even more preferably consist essentially of, HFO-1234, and even more preferably HFO-1234yf and/or HFO-1234ze in an amount of from about 5% by weight to about 50% by weight of the total heat transfer composition, more preferably from about 10% by weight to about 40% by weight, and even more preferably of from about 10% to about 30% by weight of the composition.

In preferred embodiments in which the co-refrigerant comprises fluoriodocarbon, preferably CF₃I, the composition comprises fluoriodocarbon in an amount of from about 15% by weight to about 50% by weight of the total heat transfer composition, more preferably from about 20% by weight to about 40% by weight, and even more preferably of from about 25% to about 35% by weight of the composition. In such embodiments the compounds preferably comprise, and even more preferably consist essentially of, HFO-1234,and even more preferably HFO-1234yf in an amount of from about 50% by weight to about 90% by weight of the total heat transfer composition, more preferably from about 60% by weight to about 80% by weight, and even more preferably of from about 65% to about 75% by weight of the composition.

The present uses are disclosed in connection with a wide variety of heat transfer systems in general. The preferred compositions for use in the present invention tend to exhibit many of the desirable characteristics of HFC-134a and other HFC refrigerants, including a GWP that is as low, or lower than that of conventional HFC refrigerants and a capacity that is as high or higher than such refrigerants and a capacity that is substantially similar to or substantially matches, and preferably is as high as or higher than such refrigerants. In particular, applicants have recognized that in certain preferred embodiments of the present compositions tend to exhibit relatively low global warming potentials ("GWPs"), preferably less than about 1000, more preferably less than about 500, and even more preferably less than about 150. In addition, the relatively constant boiling nature of certain of the present compositions, including the azeotrope-like compositions described in the co-pending patent applications, makes them even more desirable than certain conventional HFCs.

In certain preferred embodiments, the present compositions include, in addition to the compounds of formula I, particularly HFO-1234 (including HFO-1234ze and HFO1234yf), one or more of the following additional compounds that may be included primarily for their impact on the heat transfer characteristics, cost and the like. The following components may thus be included in the compositions as co-heat transfer fluids (or co-refrigerants in the case of cooling operations):
Trichlorofluoromethane (CFC-11)
Dichlorodifluoromethane (CFC-12)
Difluoromethane (HFC-32)
Pentafluoroethane (HFC-125)
1,1,2,2-tetrafluoroethane (HFC-134)
1,1,1,2-Tetrafluoroethane (HFC-134a)
Difluoroethane (HFC-152a)
1,1,1,2,3,3,3-Heptafluoropropane (HFC-227ea)
1,1,1,3,3,3-hexafluoropropane (HFC-236fa)
1,1,1,3,3-pentafluoropropane (HFC-245fa) 1,1,1,3,3-pentafluorobutane (HFC-365mfc) water
CO₂

The preferred heat transfer methods generally comprise providing a composition described herein and causing heat to be transferred to the composition, either by sensible heat transfer, phase change heat transfer, or a combination of these. In certain preferred embodiments, the methods for evaporative cooling, including cooling of other fluid either directly or indirectly or a body directly or indirectly, comprise condensing a refrigerant composition comprising a composition described herein and thereafter evaporating said refrigerant composition in the vicinity of the article to be cooled. As used herein, the term "body" is intended to refer not only to inanimate objects but also to living tissue, including animal tissue in general and human tissue in particular. For example, certain aspects of the present invention includes an evaporative cooling agent as defined in the attached claims to be applied to human tissue for one or more therapeutic purposes, such as a pain killing technique, as a preparatory anesthetic, or as part of a therapy involving reducing the temperature of the body being treated. The application to the body comprises providing the present compositions in liquid form under pressure, preferably in a pressurized container having a one-way discharge valve and/or nozzle, and releasing the liquid from the pressurized container by spraying or otherwise applying the composition to the body. As the liquid evaporates from the surface being sprayed, the surface cools.

In certain embodiments the present invention provides cooling by absorbing heat from a fluid or body, preferably by evaporating the present refrigerant composition in the vicinity of the body or fluid to be cooled to produce vapor comprising the present composition.

### EXAMPLES

The following examples are provided for the purpose of illustrating the present invention but without limiting the scope thereof.

### REFERENCE EXAMPLE 1

The coefficient of performance (COP) is a universally accepted measure of refrigerant performance, especially useful in representing the relative thermodynamic efficiency of a refrigerant in a specific heating or cooling cycle involving evaporation or condensation of the refrigerant. In refrigeration engineering, this term expresses the ratio of useful refrigeration to the energy applied by the compressor in compressing the vapor. The capacity of a refrigerant represents the amount of cooling or heating it provides and provides some measure of the capability of a compressor to pump quantities of heat for a given volumetric flow rate of refrigerant. In other words, given a specific compressor, a refrigerant with a higher capacity will deliver more cooling or heating power. One means for estimating COP of a refrigerant at specific operating conditions is from the thermodynamic properties of the refrigerant using standard refrigeration cycle analysis techniques (see for example, R.C. Downing, FLUOROCARBON REFRIGERANTS HANDBOOK, Chapter 3, Prentice-Hall, 1988).

A refrigeration /air conditioning cycle system is provided where the condenser temperature is about 66°C (150°F) and the evaporator temperature is about -37°C (-35°F) under nominally isentropic compression with a compressor inlet temperature of about 10°C (50°F). COP is determined for several compositions described herein over a range of condenser and evaporator temperatures and reported in Table 1 below, based upon HFC-134a having a COP value of 1.00, a capacity value of 1.00 and a discharge temperature of 79°C (175 °F).

**TABLE 1**

| REFRIGERANT COMPOSTION | Relative COP | Relative CAPACITY | DISCHARGE TEMPERATURE °C (°F) |
|---|---|---|---|
| HFO 1225ye | 1.02 | 0.76 | 70 (158) |
| HFO trans-1234ze | 1.04 | 0.70 | 74 (165) |
| HFO cis-1234ze | 1.13 | 0.36 | 68 (155) |
| HFO 1234yf | 0.98 | 1.10 | 76 (168) |

This example shows that certain of the preferred compounds for use with the present compositions each have a better energy efficiency than HFC-134a (1.02, 1.04 and 1.13 compared to 1.00) and the compressor using the present refrigerant compositions will produce discharge temperatures (70°C (158°F), 74°C (165°F) and 68°C (155°F) compared to 79°C (175°F)), which is advantageous since such result will likely leading to reduced maintenance problems. Moreover, it is evident from the above table that in one disclosure herein, namely one in which the refrigerant composition comprises, and preferably comprises at least about 70% by weight of HFO-1234yf, has a dramatically superior performance in terms of relative capacity in comparison not only to R-134a, but also to the refrigerant consisting essentially of HFO-1234ze. Therefore, provided are methods for heating or cooling an article or fluid comprising using a composition comprising at least about 80% by weight of HFO-1234yf, and even more preferably at least about 90% by weight, and in which the capacity of the refrigeration system is at least about 100%, more preferably at least about 105%, of the capacity of the same system with R-134a used as the refrigerant.

### REFERENCE EXAMPLE 2

The miscibility of HFO-1225ye and HFO-1234ze with various refrigeration lubricants is tested. The lubricants tested are mineral oil (C3), alkyl benzene (Zerol 150), ester oil (Mobil EAL 22 cc and Solest 120), polyalkylene glycol (PAG) oil (Goodwrench Refrigeration Oil for 134a systems), and a poly(alpha-olefin) oil (CP-6005- 100). For each refrigerant/oil combination, three compositions are tested, namely 5, 20 and 50 weight percent of lubricant, with the balance of each being the compound described herein being tested

The lubricant compositions are placed in heavy-walled glass tubes. The tubes are evacuated, the refrigerant compound is added, and the tubes are then sealed. The tubes are then put into an air bath environmental chamber, the temperature of which is varied from about -50°C to 70°C. At roughly 10°C intervals, visual observations of the tube contents are made for the existence of one or more liquid phases. In a case where more than one liquid phase is observed, the mixture is reported to be immiscible. In a case where there is only one liquid phase observed, the mixture is reported to be miscible. In those cases where two liquid phases were observed, but with one of the liquid phases occupying only a very small volume, the mixture is reported to be partially miscible.

The polyalkylene glycol and ester oil lubricants were judged to be miscible in all tested proportions over the entire temperature range, except that for the HFO-1225ye mixtures with polyalkylene glycol, the refrigerant mixture was found to be immiscible over the temperature range of -50°C to -30°C and to be partially miscible over from -20 to 50°C. At 50 weight percent concentration of the PAG in refrigerant and at 60°, the refrigerant/PAG mixture was miscible. At 70°C, it was miscible from 5 weight percent lubricant in refrigerant to 50 weight percent lubricants in refrigerant.

### REFERENCE EXAMPLE 3

The compatibility of the refrigerant compounds and compositions with PAG lubricating oils while in contact with metals used in refrigeration and air conditioning systems is tested at 350° C, representing conditions much more severe than are found in many refrigeration and air conditioning applications.

Aluminum, copper and steel coupons are added to heavy walled glass tubes. Two grams of oil are added to the tubes. The tubes are then evacuated and one gram of refrigerant is added. The tubes are put into an oven at 177°C (350°F) for one week and visual observations are made. At the end of the exposure period, the tubes are removed.

This procedure was done for the following combinations of oil and the compound:
a) HFC-1234ze and GM Goodwrench PAG oil
b) HFC1243 zf and GM Goodwrench oil PAG oil
c) HFC-1234ze and MOPAR-56 PAG oil
d) HFC-1243 zf and MOPAR-56 PAG oil
e) HFC-1225 ye and MOPAR-56 PAG oil.

In all cases, there is minimal change in the appearance of the contents of the tube. This indicates that the refrigerant compounds and compositions are stable in contact with aluminum, steel and copper found in refrigeration and air conditioning systems, and the types of lubricating oils that are likely to be included in such compositions or used with such compositions in these types of systems.

### COMPARATIVE EXAMPLE

Aluminum, copper and steel coupons are added to a heavy walled glass tube with mineral oil and CFC-12 and heated for one week at 350°C, as in Reference Example 3. At the end of the exposure period, the tube is removed and visual observations are made. The liquid contents are observed to turn black, indicating there is severe decomposition of the contents of the tube.

CFC-12 and mineral oil have heretofore been the combination of choice in many refrigerant systems and methods. Thus, the refrigerant compounds and compositions described herein possess significantly better stability with many commonly used lubricating oils than the widely-used prior art refrigerant-lubricating oil combination.

### REFERENCE EXAMPLE 4 - POLYOL FOAM

This example illustrates the use of blowing agent, namely the use of HFO-1234ze, and the production of polyol foams. The components of a polyol foam formulation are prepared in accordance with the following Table 2:

**TABLE 2**

| **Polyol Component** | **PBW** |
|---|---|
| Voranol 490 | 50 |
| Voranol 391 | 50 |
| Water | 0.5 |
| B-8462 (surfactant) | 2.0 |
| Polycat 8 | 0.3 |
| Polycat 41 | 3.0 |
| HFO-1234ze | 35 |
| Total | 140.8 |
| | |

| **Isocyanate** | |
|---|---|
| M-20S | 123.8 Index 1.10 |

| | |
|---|---|
| ***Voranol 490 is a sucrose-based polyol and Voranol 391 is a toluene diamine based polyol, and each are from Dow Chemical. B-8462 is a surfactant available from Degussa-Goldschmidt. Polycat catalysts are tertiary amine based and are available from Air Products. Isocyanate M-20S is a product of Bayer LLC.** | |

The foam is prepared by first mixing the ingredients thereof, but without the addition of blowing agent. Two Fisher-Porter tubes are each filled with about 52.6 grams of the polyol mixture (without blowing agent) and sealed and placed in a refrigerator to cool and form a slight vacuum. Using gas burets, about 17.4 grams of HFO-1234ze are added to each tube, and the tubes are then placed in an ultrasound bath in warm water and allowed to sit for 30 minutes. The solution produced is hazy, and a vapor pressure measurement at room temperature indicates a vapor pressure of about 584 KPa (70 psig) indicating that the blowing agent is not in solution. The tubes are then placed in a freezer at -2.8°C (27° F) for 2 hours. The vapor pressure was again measured and found to be 198 KPa (14-psig). The isocyanate mixture, about 87.9 grams, is placed into a metal container and placed in a refrigerator and allowed to cool to about 10°C (50°F). The polyol tubes were then opened and weighed into a metal mixing container (about 100 grams of polyol blend are used). The isocyanate from the cooled metal container is then immediately poured into the polyol and mixed with an air mixer with double propellers at 3000 RPM's for 10 seconds. The blend immediately begins to froth with the agitation and is then poured into an 20.3x20.3x10.2 cm (8x8x4 inch) box and allowed to foam. Because of the froth, a cream time can not be measured. The foam has a 4-minute gel time and a 5-minute tack free time. The foam is then allowed to cure for two days at room temperature.

The foam is then cut to samples suitable for measuring physical properties and is found to have a density of 34.6 kg/m³ (2.14 pcf). K-factors are measured and found to be as indicated in the following Table 3:

**TABLE 3**

| Temperature | K, mW.m⁻¹k⁻¹ (BTU In / Ft² h °F) |
|---|---|
| 4°C (40°F) | 21.1 (0.1464) |
| 24°C (75°F) | 23.7 (0.1640) |
| 43°C (110°F) | 26.1 (0.1808) |

### REFERENCE EXAMPLE 5 - POLSTYRENE FOAM

This example illustrates the use of blowing agent, namely the use of HFO-1234ze and HFO-1234yf, and the production of polystyrene foam. A testing apparatus and protocol has been established as an aid to determining whether a specific blowing agent and polymer are capable of producing a foam and the quality of the foam. Ground polymer (Dow Polystyrene 685D) and blowing agent consisting essentially of HFO-1234ze are combined in a vessel. A sketch of the vessel is illustrated below. The vessel volume is 200 cm³ and it is made from two pipe flanges and a section of 5cm (2-inch) diameter schedule 40 stainless steel pipe 10cm (4 inches) long. The vessel is placed in an oven, with temperature set at from about 88°C (190°F) to about 141°C (285°F), preferably for polystyrene at 129°C (265°F), and remains there until temperature equilibrium is reached.

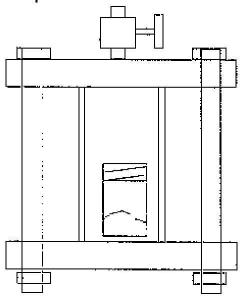

The pressure in the vessel is then released, quickly producing a foamed polymer. The blowing agent plasticizes the polymer as it dissolves into it. The resulting density of the two foams thus produced using this method are given in Table 4 and graphed in Figure 1 as the density of the foams produced using trans-HFO-1234ze and HFO-1234yf. The data show that foam polystyrene is obtainable. The die temperature for R1234ze with polystyrene is about 121°C (250°F).

**TABLE 4**

| | Dow polystyrene 685D | |
|---|---|---|
| | Foam density kg/m³ | |
| | (lb/ft³) | |
| T°C(°F) | transHFO-1234ze | HFO-1234yf |
| 135 (275) | 88.3 (55.15) | |
| 127 (260) | 3.55 (22.14) | 229 (14.27) |
| 121 (250) | 117 (7.28) | 387 (24.17) |
| 116 (240) | 271 (16.93) | |

### REFERENCE EXAMPLE 6

This example illustrates the performance of a refrigerant composition comprising HFO-1234 wherein a large proportion, and preferably at least about 75% by weight and even more preferably at least about 90% by weight, of the HFO-1234 is HFO-1234yf. More particularly, such a composition is used as a replacement for HFC-134a in four refrigerant systems. The first system is one have an evaporator temperature (ET) of about -7°C (20°F) and condenser temperature (CT) of about 54°C (130°F) (Reference Example 6A). For the purposes of convenience, such heat transfer systems, that is, systems having an ET of from about -18°C (0°F) to about 2°C (35°F) and a CT of from about 27°C (80°F) to about 54°C (130°F), are referred to herein as "medium temperature" systems. The second system is one have an ET of about -23°C (-10°F) and a CT of about 43°C (110°F) (Reference Example 6B). For the purposes of convenience, such heat transfer systems, that is, systems having an evaporator temperature of from about -29°C (-20°F) to about
-7°C (20°F) and a CT of from about 27°C (80°F) to about 54°C (130°F), are referred to herein as "refrig/freezer" systems. The third system is one have an ET of about of 2°C (35°F) and a CT of about 66°C (150°F) (Reference Example 6C). For the purposes of convenience, such heat transfer systems, that is, systems having an evaporator temperature of from about -1°C (30°F) to about 16°C (60°F) and a CT of from about 32°C (90°F) to about 93°C (200°F), are referred to herein as "automotive AC" systems. The fourth system is one have an ET of about 4°C (40°F) and a CT of about 16°C (60°F) (Reference Example 6D). For the purposes of convenience, such heat transfer systems, that is, systems having an evaporator temperature of from about 2°C (35°F) to about 10°C (50°F) and a CT of from about 27°C (80°F) to about 49°C (120°F), are referred to herein as "chiller" or "chiller AC" systems. The operation of each of such systems using R-134a and a refrigeration composition comprising at least about 90% by weight of HFO-1234yf is reported in Tables 6A - D below:

**TABLE 6A - Medium Temp Conditions -7°C (20°F) ET and 54°C (130°F) CT**

| | | R-134a | HFO-1234yf |
|---|---|---|---|
| Performance Property | Units | | |
| Capacity* | Btu/hr | 2541 | 2519 |
| Rel to R-134a | % | | 99.1% |
| COP | - | 2.31 | 2.27 |
| Rel to R-134a | % | | 98.3% |
| Discharge Press. | KPa (psig) | 1471 (198.7) | 1413 (190.3) |
| Rel to R-134a | % | | 95.8% |
| Suction Press. | KPa (psig) | 228 (18.4) | 256 (22.5) |
| Rel to R-134a | % | | 122.3% |
| Mass Flow | Kg/hr (lb/hr) | 0.31 (0.673) | 0.43 (0.958) |
| Rel to R-134a | % | | 142.3% |

| | | | |
|---|---|---|---|
| *Capacity per CFM of compressor displacement (Volumetric Capacity) | | | |

**TABLE 6B - Refrig/Freezer Temp Conditions 12°C (10°F) ET and 43°C (110°F) CT**

| | | R-134a | HFO-1234yf |
|---|---|---|---|
| Performance Property | Units | | |
| Capacity* | Btu/hr | 1234 | 1293 |
| Rel to R-134a | % | | 104.8% |
| COP | - | 1.77 | 1.71 |
| Rel to R-134a | % | | 96.6% |
| Discharge Press. | KPa (psig) | 1111 (146.4) | 1104 (145.4) |
| Rel to R-134a | % | | 99.3% |
| Suction Press. | KPa (psig) | 114 (1.9) | 143 (6.0) |
| Rel to R-134a | % | | 315.8% |
| Mass Flow | Kg/hr (lb/hr) | 0.16 (0.342) | 0.19 (0.427) |
| Rel to R-134a | % | | 124.9% |

| | | | |
|---|---|---|---|
| * Capacity per CFM of compressor displacement (Volumetric Capacity) | | | |

**TABLE 6C - Auto AC Temp Conditions 2°C (35°F) ET and 66°C (150°F) CT**

| | | R-134a | HFO-1234yf |
|---|---|---|---|
| Performance Property | Units | | |
| Capacity* | Btu/hr | 2754 | 2612 |
| Rel to R-134a | % | | 94.8% |
| COP | - | 1.91 | 1.84 |
| Rel to R-134a | % | | 96.3% |
| Discharge Press. | KPa (psig) | 1914 (262.9) | 1806 (247.3) |
| Rel to R-134a | % | | 94.1% |
| Suction Press. | KPa (psig) | 311 (30.4) | 339 (34.5) |
| Rel to R-134a | % | | 113.5% |
| Mass Flow | Kg/hr (lb/hr) | 0.40 (0.891) | 0.56 (1.235) |
| Rel to R-134a | % | | 138.6% |

| | | | |
|---|---|---|---|
| *Capacity per CFM of compressor displacement (Volumetric Capacity) | | | |

**TABLE 6D-Chiller Temp Conditions 4°C (40°F) ET and 35°C (95°F) CT**

| | | R-134a | HFO-1234yf |
|---|---|---|---|
| Performance Property | Units | | |
| Capacity* | Btu/hr | 4236 | 4060 |
| Rel to R-134a | ok | | 95.8% |
| COP | - | 6.34 | 6.23 |
| Rel to R-134a | % | | 98.3% |
| Discharge Press. | KPa (psig) | 887 (113.9) | 884 (113.5) |
| Rel to R-134a | % | | 99.6% |
| Suction Press. | KPa (psig) | 467 (35.0) | 155 (38.7) |
| Rel to R-134a | % | | 110.6% |
| Mass Flow | Kg/hr (lb/hr) | 0.47 (1.034) | 0.58 (1.268) |
| Rel to R-134a | % | | 122.6% |

| | | | |
|---|---|---|---|
| * Capacity per CFM of compressor displacement (Volumetric Capacity) | | | |

As can be seen from the Tables above, many of the important refrigeration system performance parameters are relatively close to the parameters for R-134a. Since many existing refrigeration systems have been designed for R-134a, or for other refrigerants with properties similar to R-134a, those skilled in the art will appreciate the substantial advantage of a low GWP and/or a low ozone depleting refrigerant that can be used as replacement for R-134a or like refrigerants with relatively minimal modifications to the system. Therefore, provided are retrofitting methods which comprise replacing the refrigerant in an existing system with a composition preferably comprising at least about 90% by weight and/or consists essentially of HFO-1234 and even more preferably HFO-1234yf, without substantial modification of the system. The replacement step may be a drop-in replacement in the sense that no substantial redesign of the system is required and no major item of equipment needs to be replaced in order to accommodate the refrigerant.

## Claims

1. A method for evaporative cooling,
the method comprising the steps of bringing a composition comprising 1,1,1-trifluoro-3-chloropropene (HFCO-1233zd) into contact, either directly or indirectly, with a body to be cooled and thereafter permitting the composition to evaporate or boil while in contact with said body, thereby absorbing heat from said body, wherein the composition is in liquid form and is sprayed onto the body to be cooled, wherein the method is not a method for treatment of the human or animal body by surgery or therapy.

2. The method according to claim 1, wherein the body to be cooled comprises living tissue, including animal tissue and human tissue.

3. The method according to claim 1 or 2, wherein the HFCO-1233zd is cis HFCO-1233zd, trans HFCO-1233zd or a combination thereof.

4. The method according to any preceding claim, wherein the composition comprises from about 5 % to about 99 %, preferably from about 5 % to about 95 %, by weight of HFCO-1233zd.

5. The method according to any preceding claim, wherein the composition has a Global Warming Potential (GWP) of not greater than 1000, preferably not greater than 500, and more preferably not greater than 150.

6. The method according to any preceding claim, wherein the composition has an Ozone Depleting Potential (ODP) of not greater than 0.05, preferably not greater than 0.02, more preferably of about zero.

7. The method according to any preceding claim, wherein the composition is applied to the body in liquid form under pressure.

8. The method according to claim 7, wherein the composition is applied to the body in liquid form from a pressurised container having a one-way discharge valve and/or nozzle, by spraying or otherwise applying the composition to the body.

9. An evaporative cooling agent composition comprising HFCO-1233zd for use in therapy.

10. An evaporative cooling agent composition for use as claimed in claim 9, wherein said composition is for use in killing pain in a human tissue, for use as a preparatory anaesthetic, or for use as part of a therapy involving reducing the temperature of a body to be treated.

11. An evaporative cooling agent composition for use as claimed in claim 9 or 10, wherein the HFCO-1233zd is cis HFCO-1233zd, trans HFCO-1233zd or a combination thereof.

12. An evaporative cooling agent composition for use as claimed in claim 9 to 11, wherein the composition comprises from about 5 % to about 99 %, preferably from about 5 % to about 95 %, by weight of HFCO-1233zd.

13. An evaporative cooling agent composition for use as claimed in claim 9 to 12, wherein the composition has a Global Warming Potential (GWP) of not greater than 1000, preferably not greater than 500, and more preferably not greater than 150.

14. An evaporative cooling agent composition for use as claimed in claim 9 to 12, wherein the composition has an Ozone Depleting Potential (ODP) of not greater than 0.05, preferably not greater than 0.02, more preferably of about zero.

## Patentansprüche

1. Verfahren zur Verdampfungskühlung,
bei dem man eine Zusammensetzung, die 1,1,1-trifluor-3-chlorpropen (HFCO-1233zd) umfasst, entweder direkt oder indirekt mit einem abzukühlenden Körper in Kontakt bringt und danach die Zusammensetzung in Kontakt mit dem Körper verdampfen oder sieden lässt, wodurch Wärme von dem Körper absorbiert wird, wobei die Zusammensetzung in flüssiger Form vorliegt und auf den zukühlenden Körper gesprüht wird, wobei es sich bei dem Verfahren nicht um ein Verfahren zur Behandlung des menschlichen oder tierischen Körpers durch Chirurgie oder Therapie handelt.

2. Verfahren nach Anspruch 1, bei dem der zu kühlende Körper lebendes Gewebe einschließlich tierischen Gewebes und menschlichen Gewebes umfasst.

3. Verfahren nach Anspruch 1 oder 2, wobei es sich bei dem HFCO-1233zd um cis-HFCO-1233zd, trans-HFCO-1233zd oder eine Kombination davon handelt.

4. Verfahren nach einem der vorhergehenden Ansprüche, bei dem die Zusammensetzung etwa 5 bis etwa 99 Gew.-%, vorzugsweise etwa 5 bis etwa 95 Gew.-%, HFCO-1233zd umfasst.

5. Verfahren nach einem der vorhergehenden Ansprüche, wobei die Zusammensetzung ein Treibhauspotential (Global Warming Potential, GWP) von nicht mehr als 1000, vorzugsweise nicht mehr als 500 und weiter bevorzugt nicht mehr als 150 aufweist.

6. Verfahren nach einem der vorhergehenden Ansprüche, wobei die Zusammensetzung ein Ozonschädigungspotential (Ozone Depleting Potential, ODP) von nicht mehr als 0,05, vorzugsweise nicht mehr als 0,02, weiter bevorzugt etwa null, aufweist.

7. Verfahren nach einem der vorhergehenden Ansprüche, wobei die Zusammensetzung in flüssiger Form unter Druck auf den Körper aufgebracht wird.

8. Verfahren nach Anspruch 7, wobei das Aufbringen der Zusammensetzung auf dem Körper in flüssiger Form aus einem Druckbehälter mit einem Einweg-Abgabeventil und/oder einer Einweg-Abgabedüse durch Sprühen oder anderweitiges Aufbringen der Zusammensetzung auf den Körper erfolgt.

9. Verdampfungskühlmittelzusammensetzung, umfassend HFCO-1233zd zur Verwendung bei der Therapie.

10. Verdampfungskühlmittelzusammensetzung zur Verwendung nach Anspruch 9, wobei die Zusammensetzung zur Verwendung zum Stillen von Schmerzen in einem menschlichen Gewebe, zur Verwendung als vorbereitendes Anästhetikum oder zur Verwendung als Teil einer Therapie, bei der die Temperatur eines zu behandelnden Körpers verringert wird, vorgesehen ist.

11. Verdampfungskühlmittelzusammensetzung zur Verwendung nach Anspruch 9 oder 10, wobei es sich bei dem HFCO-1233zd um cis-HFCO-1233zd, trans-HFCO-1233zd oder eine Kombination davon handelt.

12. Verdampfungskühlmittelzusammensetzung zur Verwendung nach den Ansprüchen 9 bis 11, wobei die Zusammensetzung etwa 5 bis etwa 99 Gew.-%, vorzugsweise etwa 5 bis etwa 95 Gew.-%, HFCO-1233zd umfasst.

13. Verdampfungskühlmittelzusammensetzung zur Verwendung nach den Ansprüchen 9 bis 12, wobei die Zusammensetzung ein Treibhauspotential (Global Warming Potential, GWP) von nicht mehr als 1000, vorzugsweise nicht mehr als 500 und weiter bevorzugt nicht mehr als 150 aufweist.

14. Verdampfungskühlmittelzusammensetzung zur Verwendung nach den Ansprüchen 9 bis 12, wobei die Zusammensetzung ein Ozonschädigungspotential (Ozone Depleting Potential, ODP) von nicht mehr als 0,05, vorzugsweise nicht mehr als 0,02, weiter bevorzugt etwa null, aufweist.

## Revendications

1. Procédé de refroidissement par évaporation,
le procédé comprenant les étapes consistant à mettre en contact, soit directement soit indirectement, une composition comprenant du 1,1,1-trifluoro-3-chloropropène (HFCO-1233zd) avec un corps à refroidir et par la suite laisser la composition s'évaporer ou bouillir alors qu'elle est en contact avec ledit corps, ce qui absorbe ainsi de la chaleur depuis ledit corps, dans lequel la composition est sous forme liquide et est pulvérisée sur le corps à refroidir, le procédé n'étant pas un procédé de traitement du corps humain ou animal par chirurgie ou thérapie.

2. Procédé selon la revendication 1, dans lequel le corps à refroidir comprend du tissu vivant, notamment du tissu animal et du tissu humain.

3. Procédé selon la revendication 1 ou 2, dans lequel le HFCO-1233zd est le HFCO-1233zd cis, le HFCO-1233zd trans ou une association de ceux-ci.

4. Procédé selon une quelconque revendication précédente, dans lequel la composition comprend d'environ 5 % à environ 99 %, de préférence d'environ 5 % à environ 95 %, en poids de HFCO-1233zd.

5. Procédé selon une quelconque revendication précédente, dans lequel la composition a un potentiel de réchauffement global (PRG) de pas plus de 1000, de préférence pas plus de 500 et de préférence encore pas plus de 150.

6. Procédé selon une quelconque revendication précédente, dans lequel la composition a un potentiel d'appauvrissement de la couche d'ozone (PACO) de pas plus de 0,05, de préférence pas plus de 0,02, de préférence encore d'environ zéro.

7. Procédé selon une quelconque revendication précédente, dans lequel la composition est appliquée au corps sous forme liquide sous pression.

8. Procédé selon la revendication 7, dans lequel la composition est appliquée au corps sous forme liquide à partir d'un récipient sous pression ayant une buse et/ou soupape de sortie unidirectionnelle, par pulvérisation ou autre application de la composition sur le corps.

9. Composition d'agent de refroidissement par évaporation comprenant du HFCO-1233zd destinée à être utilisée en thérapie.

10. Composition d'agent de refroidissement par évaporation destinée à être utilisée comme revendiqué dans la revendication 9, ladite composition étant destinée à être utilisée en suppression de la douleur dans un tissu humain, destinée à être utilisée en tant qu'anesthésique préparatoire ou destinée à être utilisée en tant que partie d'une thérapie impliquant la réduction de la température d'un corps à traiter.

11. Composition d'agent de refroidissement par évaporation destinée à être utilisée comme revendiqué dans la revendication 9 ou 10, dans laquelle le HFCO-1233zd est le HFCO-1233zd cis, le HFCO-1233zd trans ou une association de ceux-ci.

12. Composition d'agent de refroidissement par évaporation destinée à être utilisée comme revendiqué dans les revendications 9 à 11, la composition comprenant d'environ 5 % à environ 99 %, de préférence d'environ 5 % à environ 95 %, en poids de HFCO-1233zd.

13. Composition d'agent de refroidissement par évaporation destinée à être utilisée comme revendiqué dans les revendications 9 à 12, la composition ayant un potentiel de réchauffement global (PRG) de pas plus de 1000, de préférence pas plus de 500 et de préférence encore pas plus de 150.

14. Composition d'agent de refroidissement par évaporation destinée à être utilisée comme revendiqué dans les revendications 9 à 12, la composition ayant un potentiel d'appauvrissement de la couche d'ozone (PACO) de pas plus de 0,05, de préférence pas plus de 0,02, de préférence encore d'environ zéro.
